# EUROPEAN PATENT APPLICATION

(11) **EP 4 000 496 A1**
(43) Date of publication of application: **25.05.2022**
(21) Application number: 20858419.3
(22) Date of filing: 26.08.2020
(51) Int. Cl.: A61B 1/045

(54) **MEDICAL IMAGE PROCESSING APPARATUS AND MEDICAL OBSERVATION SYSTEM**

(30) Priority: 27.08.2019 JP 2019154630
(71) Applicant: Sony Olympus Medical Solutions Inc., Hachioji-shi, Tokyo 192-0904 (JP)
(72) Inventor: SUGISAKI, Kiminori, Hachioji-shi, Tokyo 192-0904 (JP); USHIRODA, Hiroshi, Hachioji-shi, Tokyo 192-0904 (JP)
(74) Representative: D Young & Co LLP
(86) International application number: PCT/JP2020/032251
(87) International publication number: WO 2021/039870

(57) **Abstract**

Provided are a medical image processing device and a medical observation system that are configured to clarify a boundary between a near-infrared image and a visible image in a specific region. An image processing unit 93 uses a first image based on first image data input from outside and a second image based on second image data input from outside, having an enhanced specific region of the first image, and generates third image data in which the specific region of the first image is replaced with mixed image data having a mixture of a group of first pixels located in the specific region of the first image and a group of second pixels located in the specific region of the second image.

## Description

### Field

The present disclosure relates to a medical image processing device and a medical observation system that perform image processing on image data input from outside. Background

In medical and industrial cameras, there is known a technique of generating a synthetic image by adding a near-infrared image showing a specific part acquired by capturing an optical image in a near-infrared light band by first imaging means, and a visible image acquired by capturing an optical image of a visible light region by second imaging means, at a predetermined ratio for each pixel (e.g., see Patent Literature 1).

### Citation List

### Patent Literature

Patent Literature 1: JP 2015-29841 A

### Summary

### Technical Problem

However, in Patent Literature 1 described above, the near-infrared image and the visible image are added at the predetermined ratio for each pixel. Therefore, there is a problem that information about a background visible image in the specific region showing the specific part is lost, making a boundary between the near-infrared image and the visible image in a specific region unclear.

The present disclosure has been made in view of the above description, and an object of the present disclosure is to provide a medical image processing device and a medical observation system that are configured to clarify a boundary between a near-infrared image and a visible image in a specific region.

### Solution to Problem

To solve the above-described problem and achieve the object, a medical image processing device according to the present disclosure includes an image processor configured to: use a first image based on first image data input from outside, and a second image based on second image data input from outside, the second image data including an enhanced specific region of the first image, and generate third image data in which the specific region of the first image is replaced with mixed image data including a mixture of a group of first pixels located in the specific region of the first image and a group of second pixels located in the specific region of the second image.

Moreover, in the medical image processing device according to the present disclosure, the image processor is configured to generate the third image data by regularly arranging the group of first pixels and the group of second pixels in the specific region.

Moreover, in the medical image processing device according to the present disclosure, the image processor is configured to generate the third image data by alternately arranging pixels of the group of first pixels and the group of second pixels, on horizontal lines in the specific region.

Moreover, in the medical image processing device according to the present disclosure, the image processor is configured to generate the third image data by arranging pixels of the group of first pixels and the group of second pixels in a lattice pattern in the specific region.

Moreover, in the medical image processing device according to the present disclosure, the image processor is configured to generate the third image data by alternately arranging the group of first pixels and the group of second pixels for each horizontal line or each vertical line in the specific region.

Moreover, in the medical image processing device according to the present disclosure, the image processor is configured to generate the third image data by changing a display area of a second pixel in a mixed image based on the mixed image data according to a luminance value of each of the groups of second pixels.

Moreover, in the medical image processing device according to the present disclosure, the image processor is configured to generate the third image data by irregularly arranging the group of first pixels and the group of second pixels in the specific region.

Moreover, in the medical image processing device according to the present disclosure, the image processor is configured to generate the third image data by converting color of the group of second pixels into a color to enhance the group of second pixels.

Moreover, in the medical image processing device according to the present disclosure, the image processor is configured to generate the third image data by converting color of each of the groups of second pixels into a color enhancing each of the groups of second pixels while maintaining a luminance value thereof.

Moreover, in the medical image processing device according to the present disclosure, the image processor is configured to generate the third image data by converting color of each of the groups of second pixels into a color according to a luminance value thereof.

Moreover, in the medical image processing device according to the present disclosure, the image processor is configured to generate, for each pixel, synthetic data in which the first image data of each pixel of the group of first pixels is blended with the second image data of each pixel of the group of second pixels, as display data of each pixel of the group of second pixels.

Moreover, in the medical image processing device according to the present disclosure, the synthetic data is generated by blending using data information of at least one of hue, luminance, and saturation of each pixel of the first image and second image.

Moreover, in the medical image processing device according to the present disclosure, the specific region is a light-emitting region that emits light in response to irradiation of a fluorescent substance with excitation light.

Moreover, a medical observation system according to the present disclosure includes: the medical image processing device according to the present disclosure; a light source device configured to emit white light and near-infrared light to a subject where a fluorescent substance is sprayed; and an observation device configured to generate the first image data by receiving reflection light from the subject in a case where the white light is emitted, and generate the second image data by receiving light emitted from the fluorescent substance when the near-infrared light is emitted, wherein the medical image processing device is configured to acquire the first image data and the second image data from the observation device. Advantageous Effects of Invention

According to the present disclosure, the boundary between the specific region and the visible image can be effectively made clear.

### Brief Description of Drawings

FIG. 1 is a diagram illustrating a schematic configuration of an endoscope system according to a first embodiment.
FIG. 2 is a block diagram illustrating functional configurations of a light source device, a camera head, and a control device included in the endoscope system according to the first embodiment.
FIG. 3 is a flowchart illustrating the outline of a process performed by an endoscope system 1 according to the first embodiment.
FIG. 4 is a view schematically illustrating an example of a first image generated by an imaging unit.
FIG. 5 is a view schematically illustrating an example of a second image generated by the imaging unit.
FIG. 6 is a view schematically illustrating an example of a third image generated by an image processing unit.
FIG. 7 is an enlarged view of a part of a feature area of FIG. 6.
FIG. 8 is an enlarged view of a part of a feature area of a third image generated by an image processing unit according to a first modification of the first embodiment.
FIG. 9 is an enlarged view of a part of a feature area of a third image generated by an image processing unit according to a second modification of the first embodiment.
FIG. 10 is a diagram schematically illustrating a method of generating a third image to be generated by an image processing unit according to a third modification of the first embodiment.
FIG. 11 is a diagram illustrating a schematic configuration of an endoscope system according to a second embodiment.
FIG. 12 is a diagram illustrating a schematic configuration of a surgical microscope system according to a third embodiment.

### Description of Embodiments

Modes for carrying out the present disclosure will be described below in detail with reference to the drawings. Note that the present disclosure is not limited to the following embodiments. In addition, the drawings referred to in the following descriptions are merely schematically illustrated in shape, size, and positional relationship to the extent of understanding the contents of the present disclosure. In other words, the present disclosure is not limited only to the shapes, sizes, and positional relationships exemplified in the drawings. Furthermore, in the drawings, the same portions are denoted by the same reference signs for description. Furthermore, as an example of the medical observation system according to the present disclosure, an endoscope system including a rigid endoscope will be described.

### (First embodiment)

### [Outline of configuration of endoscope system]

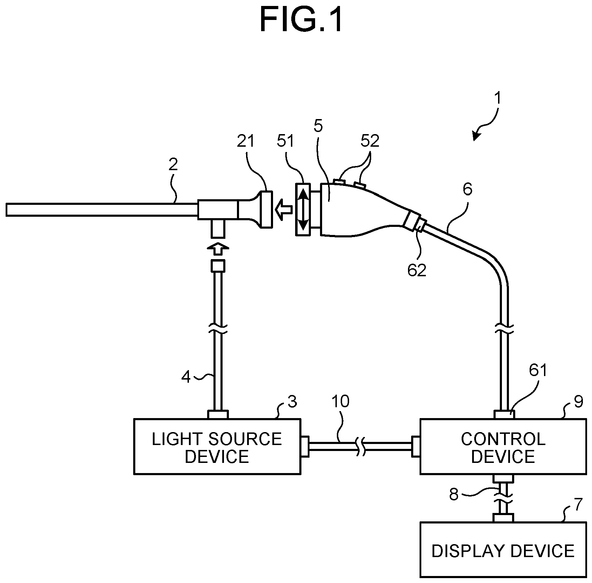

FIG. 1 is a diagram illustrating a schematic configuration of an endoscope system according to a first embodiment. The endoscope system 1 illustrated in FIG. 1 is a system that is used in a medical field, is inserted into a living body of a subject, such as a living body of a human or animal, and observes the subject by displaying an image obtained by imaging the inside thereof. Note that, in the first embodiment, a rigid endoscope system using a rigid endoscope (insertion section 2) illustrated in FIG. 1 will be described as the endoscope system 1, but the present invention is not limited thereto, and, for example, a flexible endoscope system may be used.

The endoscope system 1 illustrated in FIG. 1 includes the insertion section 2, a light source device 3, a light guide 4, a camera head 5 (endoscopic imaging device), a first transmission cable 6, a display device 7, a second transmission cable 8, a control device 9, and a third transmission cable 10.

The insertion section 2 is rigid or at least partially flexible and has an elongated shape. The insertion section 2 is inserted into the subject such as a patient. The insertion section 2 internally includes one or a plurality of lenses, and is provided with an optical system that combines an observation image.

One end of the light guide 4 is connected to the light source device 3. Under the control of the control device 9, the light source device 3 emits (supplies), to one end of the light guide 4, white light for illuminating inside the subject and excitation light or infrared light for a drug administered or sprayed to the subject. The light source device 3 includes a light emitting diode (LED) light source or a semiconductor laser element such as a laser diode (LD). The light source device 3 and the control device 9 may be configured to communicate individually as illustrated in FIG. 1, or may configured to be integrated with each other.

The light guide 4 has one end that is detachably connected to the light source device 3 and has the other end that is detachably connected to the insertion section 2. The light guide 4 guides light emitted from the light source device 3 from the one end to the other end and supplies the light to the insertion section 2.

To the camera head 5, an eyepiece 21 of the insertion section 2 is detachably connected. Under the control of the control device 9, the camera head 5 generates image data (imaging signal) by capturing the observation image formed by the insertion section 2, and outputs the image data. In addition, the camera head 5 includes an operation ring unit 51 that is provided to be turnable in the circumferential direction, and a plurality of input units 52 that receives input of instruction signals giving instructions for various operations of the endoscope system 1.

The first transmission cable 6 has one end that is detachably connected to the control device 9 via a first connector 61, and the other end that is connected to the camera head 5 via a second connector 62. The first transmission cable 6 transmits the image data output from the camera head 5, to the control device 9, and transmits a control signal, synchronization signal, clock signal, power, or the like output from the control device 9, to the camera head 5.

The display device 7 is configured to be connected to the control device 9 via the second transmission cable 8, and displays a display image based on the image data processed by the control device 9 under the control of the control device 9.

The second transmission cable 8 has one end that is detachably connected to the display device 7, and the other end that is detachably connected to the control device 9. The second transmission cable 8 transmits the display image based on the image data processed by the control device 9, to the display device 7.

The control device 9 includes a memory and a processor having hardware such as a central processing unit (CPU), a graphics processing unit (GPU), an application specific integrated circuit (ASIC), and a field programmable gate array (FPGA). The control device 9 collectively controls operations of the light source device 3, the camera head 5, and the display device 7 via the first transmission cable 6, the second transmission cable 8, and the third transmission cable 10, according to programs recorded in the memory. In addition, the control device 9 performs various types of image processing on the image data input from the camera head 5 via the first transmission cable 6, and outputs the image data to the second transmission cable 8.

The third transmission cable 10 has one end that is detachably connected to the light source device 3, and the other end that is detachably connected to the control device 9. The third transmission cable 10 transmits the control signal transmitted from the control device 9 to the light source device 3.

### [Detailed configurations of light source device, camera head, and control device]

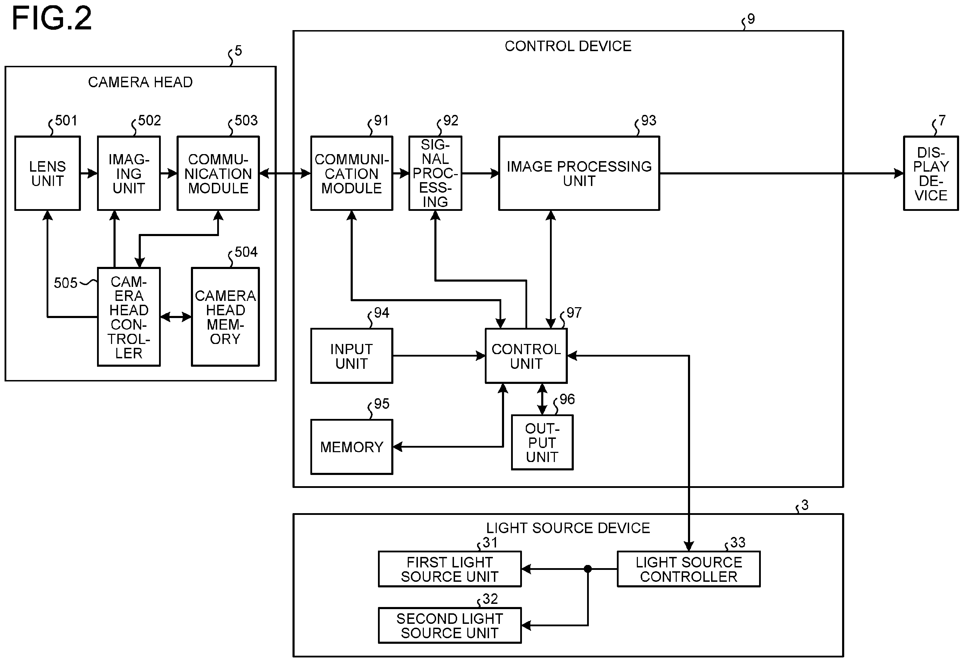

Next, the functional configurations of the light source device 3, the camera head 5, and the control device 9 will be described. FIG. 2 is a block diagram illustrating functional configurations of the light source device 3, the camera head 5, and the control device 9 included in the endoscope system 1. Note that in FIG. 2, the insertion section 2, the light guide 4, the first transmission cable 6, the second transmission cable 8, and the third transmission cable 10 are omitted for convenience of description.

### [Configuration of light source device]

The configuration of the light source device 3 will be described first.

The light source device 3 includes a first light source unit 31, a second light source unit 32, and a light source controller 33.

In the first light source unit 31, under the control of the light source controller 33, white light pulses are generated, and the white light to be emitted to the subject is supplied to the insertion section 2. The first light source unit 31 includes a red semiconductor laser element that is configured to emit red light (a wavelength band of 600 nm to 700 nm), a blue semiconductor laser element that is configured to emit blue light (a wavelength band of 400 nm to 500 nm), and a green semiconductor laser element that is configured to emit green light (a wavelength band of 500 nm to 600 nm). Note that the first light source unit 31 includes, but is not limited to, the red, blue, and green semiconductor laser elements, and a white semiconductor laser element that is configured to emit white light may be used. Furthermore, the first light source unit 31 does not need to be the semiconductor laser element, and may employ, for example, a light emitting diode (LED) or the like. Furthermore, the first light source unit 31 is not limited to simultaneous lighting for simultaneously lighting with the red light, green light, and blue light, and may be sequential lighting for sequentially lighting with the red light, green light, and blue light.

Under the control of the light source controller 33, the second light source unit 32, generates infrared light pulses to be emitted to the subject via the insertion section 2. Specifically, under the control of the light source controller 33, the second light source unit 32 emits infrared light (a wavelength band of 700 to 1000 nm) that excites the drug (fluorescent substance) introduced into the subject, and supplies the infrared light to the insertion section 2. The second light source unit 32 includes a semiconductor laser element that is configured to emit light (700 to 1000 nm) exciting the fluorescent substance, a filter that transmits only a predetermined wavelength band, and the like. Note that in the following description, light emitted from the second light source unit 32 is the infrared light, but is not limited thereto. For example, the light may be light (a wavelength band near 405 nm) used for photo dynamic diagnosis (PDD) observation for observation of fluorescence of a photosensitive substance, such as hematoporphyrin derivative, accumulated in tumor tissue in advance, light (a wavelength band of 390 to 470 nm + a wavelength band of 540 to 560 nm) used for auto fluorescence imaging (AFI) observation for observation of auto fluorescence from a fluorescent substance, such as collagen, or the like.

The light source controller 33 controls light emission from the first light source unit 31 and the second light source unit 32, under the control of the control device 9. The light source controller 33 includes a memory and a processor having hardware such as a CPU, ASIC, and

### FPGA.

### [Configuration of camera head]

Next, a configuration of the camera head 5 will be described.

The camera head 5 includes a lens unit 501, an imaging unit 502, a communication module 503, a camera head memory 504, and a camera head controller 505.

The lens unit 501 includes one or a plurality of lenses, and forms an object image on a light receiving surface of the imaging unit 502. Furthermore, in the lens unit 501, under the control of the camera head controller 505, a drive unit, which is not illustrated, moves the lenses in an optical axis direction to provide auto focus (AF) for changing the focal position and optical zoom for changing the focal length. Note that in the first embodiment, a diaphragm mechanism may be provided in the lens unit 501 and a removable optical filter mechanism may be provided on the optical axis.

Under the control of the camera head controller 505, the imaging unit 502 (image sensor) receives light of the object image formed by the insertion section 2 and the lens unit 501, performs photoelectric conversion to generate image data (raw data), and outputs the image data to the communication module 503. Specifically, the imaging unit 502 outputs, to the communication module 503, a first image (hereinafter, simply referred to as a "first image") based on first image data generated by imaging, upon irradiating the subject by the first light source unit 31. Furthermore, the imaging unit 502 outputs, to the communication module 503, a second image (hereinafter, simply referred to as a "second image") based on second image data generated by imaging, in a special observation mode in which the second light source unit 32 irradiates the drug administered to the subject with infrared light to perform imaging. Here, the second image is an image having an enhanced specific region in an observation field substantially the same as that of the first image. Furthermore, the specific region is a region of the subject to which the drug containing a fluorescent substance is administered. The imaging unit 502 includes a charge coupled device (CCD), complementary metal oxide semiconductor (CMOS), or the like.

The communication module 503 outputs various signals transmitted from the control device 9 via the first transmission cable 6, to each unit in the camera head 5. Furthermore, the communication module 503 performs parallel/serial conversion processing or the like on information about the first image and second image generated by the imaging unit 502, information about the current state of the camera head 5, and the like via the first transmission cable 6, and outputs the information to the control device 9.

The camera head memory 504 stores camera head information for identification of the camera head 5 and various programs executed by the camera head 5. Here, the camera head information includes the number of pixels and a pixel pitch of the imaging unit 502, an identification ID of the camera head 5, and the like. The camera head memory 504 uses a volatile memory, a non-volatile memory, and the like.

The camera head controller 505 controls the operations of the units constituting the camera head 5, on the basis of various signals input from the communication module 503. The camera head controller 505 includes a memory and a processor having hardware such as a CPU.

### [Configuration of control device]

Next, the configuration of the control device 9 will be described.

The control device 9 includes a communication module 91, a signal processing unit 92, an image processing unit 93, an input unit 94, a memory 95, an output unit 96, and a control unit 97.

The communication module 91 outputs various signals including the imaging signal input from the camera head 5 to the control unit 97 and the signal processing unit 92. Furthermore, the communication module 91 transmits various signals input from the control unit 97 to the camera head 5. Specifically, the communication module 91 performs parallel/serial conversion processing and the like on the signals input from the control unit 97 and outputs the signals to the camera head 5. Furthermore, the communication module 91 performs serial/parallel conversion processing and the like on the signals input from the camera head 5 and outputs the signals to the units constituting the control device 9.

The signal processing unit 92 performs signal processing such as noise reduction processing or A/D conversion processing on the first image or the second image input from the camera head 5 via the communication module 91, and outputs the first image or the second image to the image processing unit 93.

Under the control of the control unit 97, the image processing unit 93 performs various types of image processing on the first image or the second image input from the signal processing unit 92, and outputs the first image or the second image to the display device 7. Here, predetermined image processing includes various types of known image processing such as interpolation processing, color correction processing, color enhancement processing, and contour enhancement processing. Furthermore, the image processing unit 93 generates a third image (hereinafter, simply referred to as a "third image") based on third image data in which the specific region of the first image is replaced with mixed image data, and outputs the third image to the display device 7. The mixed image data has a mixture of a group of first pixels located in the specific region of the first image and a group of second pixels located in the specific region of the second image. In addition, the image processing unit 93 generates the third image by alternately arranging pixels of the group of first pixels located in the specific region of the first image and pixels of the group of second pixels located in the specific region of the second image, on horizontal lines in a feature area. Specifically, the image processing unit 93 generates the third image by replacing first pixels located in the specific region of the first image with second pixels located in the specific region of the second image at predetermined intervals, on the horizontal lines of the feature area. The image processing unit 93 includes a memory and a processor having hardware such as a GPU, FPGA, or CPU. Note that, in the first embodiment, the image processing unit 93 functions as a medical image processing device.

The input unit 94 includes a keyboard, a mouse, a touch panel, and the like. The input unit 94 receives various types of information input through user's operations.

The memory 95 uses a volatile memory, a non-volatile memory, a frame memory, and the like. The memory 95 stores various programs executed by the endoscope system 1 and various data used during processing. Note that the memory 95 may further include a memory card or the like that is attachable to the control device 9.

The output unit 96 includes a speaker, a printer, a display, and the like. The output unit 96 outputs various types of information about the endoscope system 1.

The control unit 97 collectively controls the units constituting the endoscope system 1. The control unit 97 includes hardware such as a memory and a CPU.

### [Process by endoscope system]

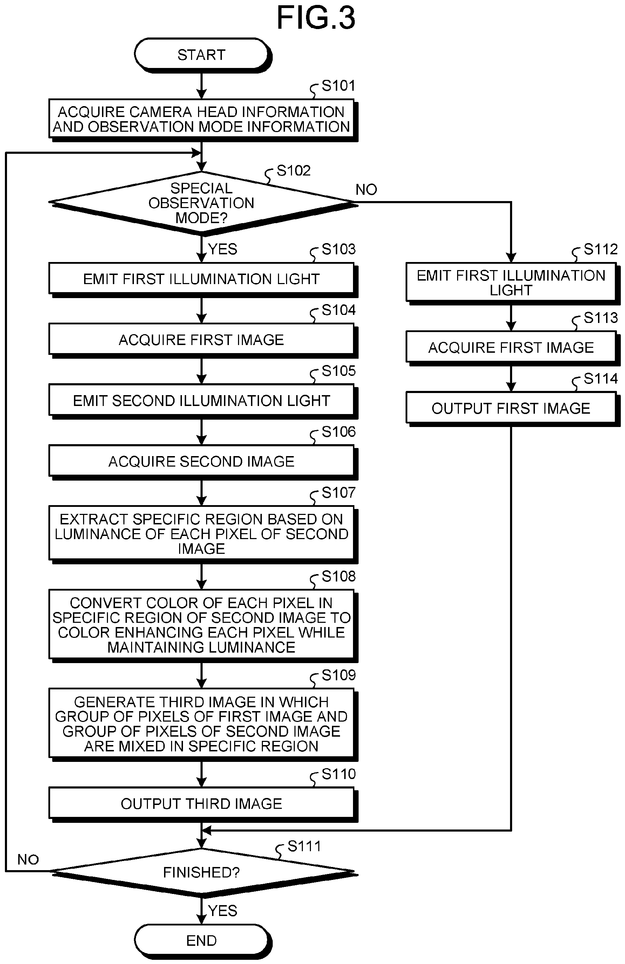

Next, a process performed by the endoscope system 1 will be described. FIG. 3 is a flowchart illustrating the outline of the process performed by the endoscope system 1.

As illustrated in FIG. 3, the control unit 97 acquires the camera head information from the camera head 5 via the communication module 91 and observation mode information indicating a current observation mode of the endoscope system 1 from the memory 95, first (Step S101).

Subsequently, the control unit 97 determines whether the endoscope system 1 is in the special observation mode, on the basis of the observation mode information acquired from the memory 95 (Step S102). When the control unit 97 determines that the endoscope system 1 is in the special observation mode (Step S102: Yes), the endoscope system 1 proceeds to Step S103 which is described later. On the other hand, when the control unit 97 determines that the endoscope system 1 is not in the special observation mode (Step S102: No), the endoscope system 1 proceeds to Step S112 which is described later.

In Step S103, the control unit 97 causes the light source device 3 to emit white light as first illumination light. In this configuration, the imaging unit 502 receives reflection light of the white light reflected from the object via the lens unit 501, and performs photoelectric conversion to generate a first image P1.

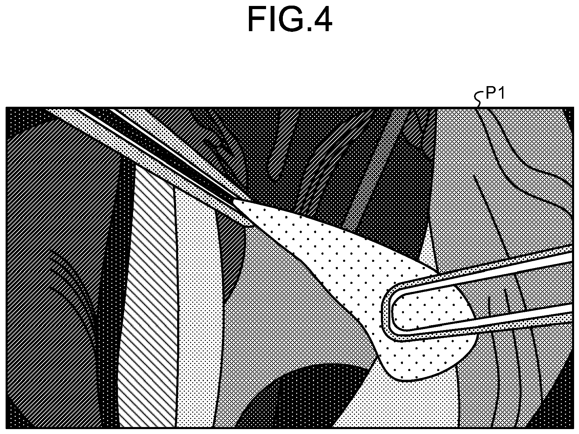

Subsequently, under the control of the control unit 97, the image processing unit 93 acquires the first image from the imaging unit 502 via the communication module 503, the communication module 91, and the signal processing unit 92 (Step S104). For example, the image processing unit 93 acquires the first image P1 as illustrated in FIG. 4.

Thereafter, the control unit 97 causes the light source device 3 to emit near-infrared light as second illumination light (Step S105). In this configuration, the imaging unit 502 receives light emitted from the fluorescent substance applied to the object via the lens unit 501, and performs photoelectric conversion to generate the second image.

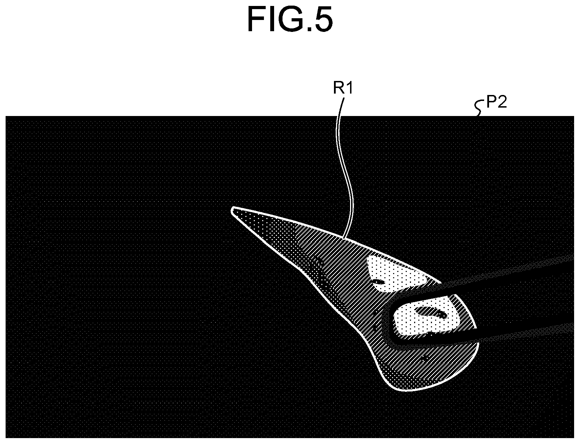

Subsequently, under the control of the control unit 97, the image processing unit 93 acquires the second image from the imaging unit 502 via the communication module 503, the communication module 91, and the signal processing unit 92 (Step S106). For example, the image processing unit 93 acquires a second image P2 as illustrated in FIG. 5. The second image P2 includes a light-emitting region in which the fluorescent substance emits light. The light-emitting region is a specific region R1 in an observation region substantially the same as that of the first image P1.

Then, the image processing unit 93 extracts the feature area on the basis of a luminance value of each pixel in the second image (Step S107). Specifically, the image processing unit 93 determines whether the luminance value is equal to or larger than a predetermined threshold for each pixel constituting the second image, and extracts, as the specific region, pixels having luminance equal to or larger than the predetermined threshold from the second image. For example, in the second image P2 as illustrated in FIG. 5, the image processing unit 93 extracts, as the specific region R1, pixels in a region where the pixels have luminance equal to or larger than the predetermined threshold. Here, the value equal to or larger than the predetermined threshold is a value that can distinguish luminance upon light emission of the fluorescent substance from noise generated in the imaging unit 502. Furthermore, the noise includes random noise, blinking defect noise, pixel defect noise, thermal noise, and the like. Note that the predetermined threshold may be calculated by calibration processing or the like of obtaining an average luminance value of a plurality of images generated by causing the imaging unit 502 to capture a plurality of images in a state where the lens unit 501 is shielded in advance so as to cause the camera head memory 504 to record this calculation result. As a matter of course, as the predetermined threshold, a threshold corresponding to the camera head ID recorded in the server may be acquired via a network.

Next, the image processing unit 93 converts the color of each pixel in the specific region into a color enhancing the pixel, while maintaining the luminance value of each pixel in the feature area of the second image (Step S108). Specifically, the image processing unit 93 converts the luminance of each pixel in the feature area extracted from the second image into a color difference. For example, the image processing unit 93 converts the luminance of each pixel in the feature area extracted from the second image into a YCbCr format to convert the luminance into color difference information, and enhances the pixel group in the feature area of the second image.

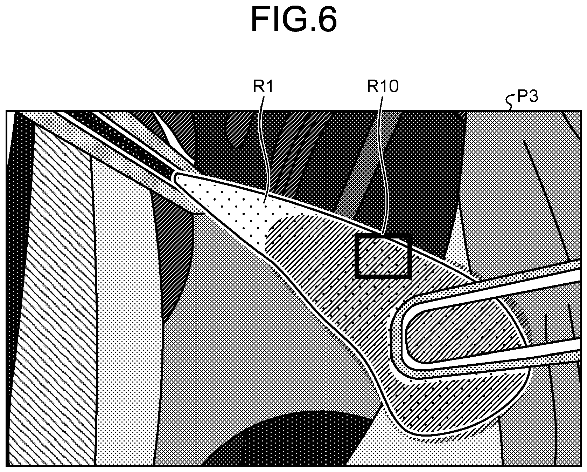

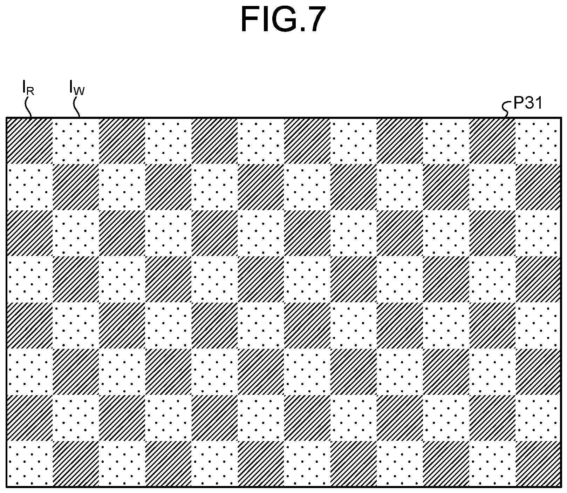

Thereafter, the image processing unit 93 generates the third image in which the pixel group of the first image in the feature area and the pixel group of the second image in the specific region are mixed (Step S109), and outputs the third image to the display device 7 (Step S110). Specifically, as illustrated in FIG. 6, the image processing unit 93 generates a third image P3 in which the group of first pixels of the first image and the group of second pixels of the second image are mixed in the feature area. FIG. 7 is an enlarged view of a portion R10 of the feature area of FIG. 6. As illustrated in an image P31 of FIG. 7, the image processing unit 93 generates the third image P3 (see FIG. 6) by mixing a group of first pixels IW of the first pixels in the specific region R1 of the first image and a group of second pixels IR in the specific region R1 of the second image. More specifically, the image processing unit 93 alternately and regularly replaces first pixels IW in the feature area of the first image with second pixels IR of the second image on the horizontal lines of the feature area, and generates the third image P3 in which the group of second pixels IR is mixed in the specific region of the first image. For example, as illustrated in FIG. 7, the image processing unit 93 regularly replaces the group of first pixels IW of the first pixels in the feature area of the first image with the group of second pixels IR of the second image into a staggered lattice pattern, and generates the third image P3. This configuration makes it possible to make the boundary between the second image (near-infrared image) and the first image (visible image) in the specific region clear, while maintaining the information of the visible image that is the first image indicating background. Note that, in FIG. 7, the image processing unit 93 alternately and regularly replaces the first pixels IW of the first pixels with the second pixels IR of the second image in the feature area into the lattice pattern. However, for example, the first pixels IW may be replaced with the second pixels IR located in the specific region R1 of the second image at two-pixel intervals, and the first pixels IW can be appropriately changed.

Subsequently, when the instruction signal giving instruction for finish of the observation of the subject is input from the input unit 94 (Step S111: Yes), the endoscope system 1 ends the present processing. On the other hand, when no instruction signal giving instruction for finish of the observation of the subject is input from the input unit 94 (Step S111: No), the endoscope system 1 returns to Step S102 described above.

In Step S112, the control unit 97 causes the light source device 3 to emit white light as the first illumination light. In this configuration, the imaging unit 502 receives reflection light of the white light reflected from the object via the lens unit 501, and performs photoelectric conversion to generate the first image P1.

Subsequently, under the control of the control unit 97, the image processing unit 93 acquires the first image from the imaging unit 502 via the communication module 503, the communication module 91, and the signal processing unit 92 (Step S113), performs the predetermined image processing on the first image, and outputs the first image to the display device 7 (Step S114). After Step S114, the endoscope system 1 proceeds to Step S111.

According to the first embodiment described above, the image processing unit 93 generates the third image in which the specific region of the first image is replaced with the mixed image data having a mixture of the group of first pixels located in the specific region of the first image and the group of second pixels located in the specific region of the second image. Therefore, it is possible to make the boundary between the second image and the first image in the specific region clear, while maintaining the information about the visible image as the first image indicating the background.

Furthermore, according to the first embodiment, the image processing unit 93 generates the third image by regularly arranging the group of first pixels and the group of second pixels in the feature area of the first image. Therefore, it is possible to identify a portion of the specific region where fluorescence is generated by the infrared light being special light while leaving a portion of the visible image as a white image, making it possible to easily grasp the position of a lesion area.

In addition, according to the first embodiment, the image processing unit 93 generates the third image by alternately arranging the first pixels located in the feature area and the second pixels located in the feature area on the horizontal lines in the feature area of the first image. Therefore, it is possible to easily grasp the position of the lesion area, improving operability in surgical processing.

Note that, in the first embodiment, the image processing unit 93 regularly arranges the group of first pixels of the first image located in the feature area and the group of second pixels of the second image located in the feature area, in the feature area of the first image. However, the image processing unit 93 may generate the third image by irregularly arranging the group of first pixels of the first image located in the feature area and the group of second pixels of the second image located in the feature area.

### (First modification of first embodiment)

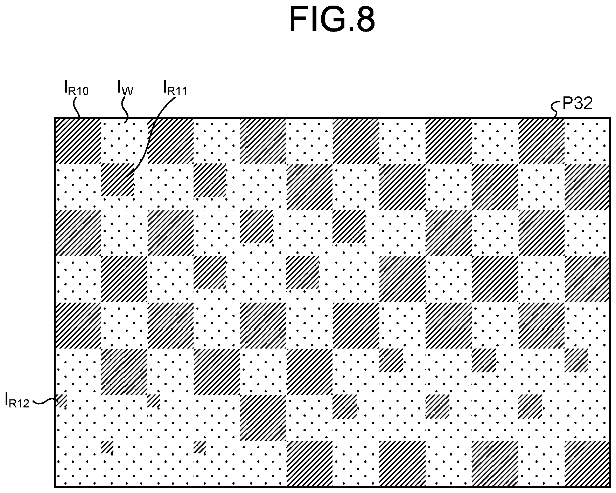

Next, a first modification of the first embodiment will be described. FIG. 8 is an enlarged view of a part of a feature area of the third image generated by the image processing unit according to the first modification of the first embodiment.

As illustrated in an image P32 of FIG. 8, the image processing unit 93 generates the third image in which the first pixels IW of the first pixels and the second pixels IR of the second image are mixed in the feature area of the first image. In this configuration, as illustrated in FIG. 8, the image processing unit 93 changes the display area of each of the second pixels, on the basis of the luminance value of each pixel in the feature area of the second image. Then, the image processing unit 93 generates the third image by replacing the first pixels IW located in the feature area of the first image with a second pixel IR10, a second pixel IR11, and a second pixel IR12 whose display areas are changed. Note that in FIG. 8, the display area of the second pixel according to the luminance value of each pixel in the feature area of the second image is represented by changing the display area which is hatched. Furthermore, in FIG. 8, the larger the luminance value, the larger the display area (second pixel IR10 > second pixel IR11 > second pixel IR12).

According to the first modification of the first embodiment described above, the image processing unit 93 changes the display area of the second pixel on the basis of the luminance value of each pixel in the feature area of the second image, and generates the third image by replacing the first pixels IW with the second pixels IR having changed display areas. Therefore, it is possible to identify a portion of the specific region where fluorescence is generated by the infrared light being special light while leaving a portion of the visible image as the white image, making it possible to easily grasp the position of the lesion area.

### (Second modification of first embodiment)

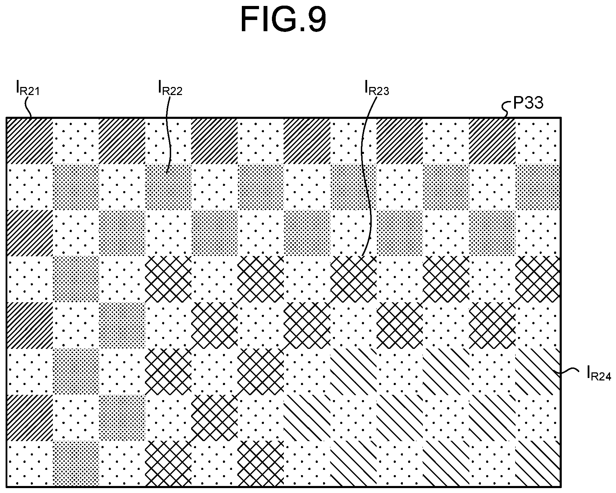

Next, a second modification of the first embodiment will be described. FIG. 9 is an enlarged view of a part of a feature area of the third image generated by the image processing unit according to the second modification of the first embodiment.

As illustrated in an image P33 of FIG. 9, the image processing unit 93 generates the third image in which the first pixels IW and the second pixels IR of the second image are mixed in the feature area of the first image. In this configuration, the image processing unit 93 changes the color of each second pixel IR on the basis of the luminance value of each pixel in the feature area of the second image. Then, the image processing unit 93 generates the third image by replacing second pixel IR21 to second pixel IR24 that have display colors changed according to the luminance value with the first pixels IW. For example, the image processing unit 93 enhances the color as the luminance value is larger (second pixel IR21 > second pixel IR22 > second pixel IR23 > second pixel IR24). For example, the image processing unit 93 converts the second pixel IR21 to green color, the second pixel IR22 to blue color, the second pixel IR23 to magenta color, and the second pixel IR24 to red color.

According to the second modification of the first embodiment described above, the image processing unit 93 changes the color of the second pixel IR on the basis of the luminance value of each pixel in the feature area of the second image, and generates the third image by replacing the second pixels IR1 to IR4 whose display colors have been changed according to the luminance value, with the first pixels IW. Therefore, it is possible to identify a portion of the specific region where fluorescence is generated by the infrared light being special light, making it possible to easily grasp the position of the lesion area.

### (Third modification of first embodiment)

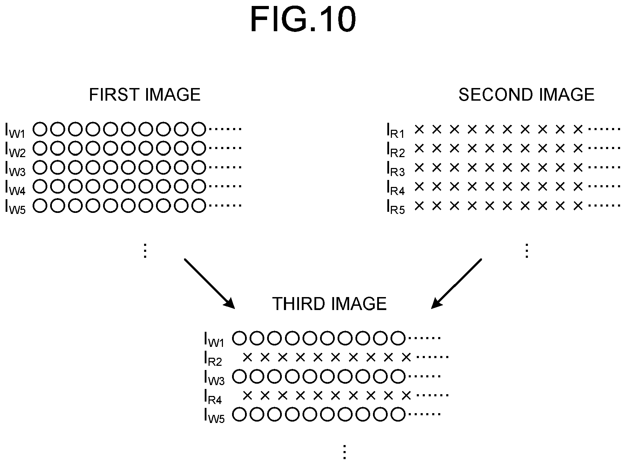

Next, a third modification of the first embodiment will be described. FIG. 10 is a diagram schematically illustrating a method of generating the third image to be generated by the image processing unit 93 according to the third modification of the first embodiment.

As illustrated in FIG. 10, the image processing unit 93 generates the third image by alternately arranging the group of first pixels in the feature area of the first image and the group of second images in the feature area of the second image, for each horizontal line. Specifically, the image processing unit 93 generates the third image by replacing a group of first pixels IW2 and a group of first pixels IW4 on even-numbered horizontal lines in the feature area of the first image with a group of second pixels IR2 and a group of second pixels IR4 on even-numbered horizontal lines in the feature area of the second image.

According to the third modification of the first embodiment described above, the image processing unit 93 generates the third image by replacing the group of first pixels IW2 and the group of first pixels IW4 on the even-numbered horizontal lines in the feature area of the first image with the group of second pixels IR2 and the group of second pixels IR4 of the second image on the even-numbered horizontal lines in the feature area of the second image. Therefore, it is possible to identify a portion of the specific region where fluorescence is generated by the infrared light being special light, making it possible to easily grasp the position of the lesion area.

Note that, in the third modification of the first embodiment described above, the image processing unit 93 may generate the third image by alternately arranging the group of first pixels in the feature area of the first image and the group of second images in the feature area of the second image, for each vertical line.

Furthermore, in the third modification of the first embodiment described above, the image processing unit 93 generates the third image by mixing the group of first pixels and the group of second pixels, for each line in the feature area. However, the third image may be generated by mixing the group of first images and the group of second pixels for every predetermined lines, for example, for every two lines, or the third image may be generated by mixing the group of first images and the group of second pixels for odd-numbered or even-numbered horizontal or vertical lines.

### (Second embodiment)

Next, a second embodiment will be described. In the first embodiment described above, a description has been made of application to the rigid endoscope system using the rigid endoscope, but in the second embodiment, application to a flexible endoscope system using a flexible endoscope will be described. Note that the same configurations as those of the endoscope system 1 according to the first embodiment described above are denoted by the same reference signs, and detailed description thereof will be omitted.

### [Outline of configuration of endoscope system]

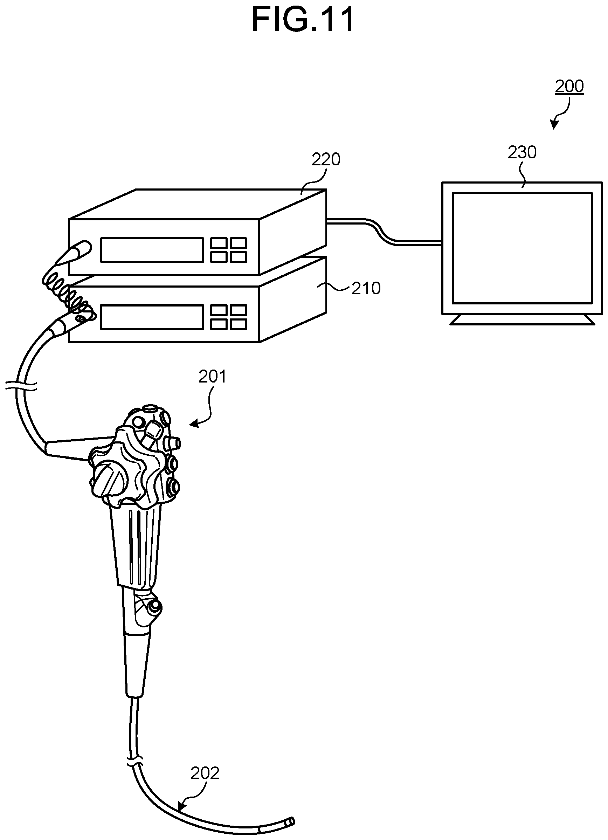

FIG. 11 is a diagram illustrating a schematic configuration of an endoscope system according to the second embodiment. An endoscope system 200 illustrated in FIG. 11 includes an endoscope 201 that captures an in-vivo image of an observed region by an insertion section 202 inserted into the subject and generates image data, a light source device 210 that supplies white light or infrared light to the endoscope 201, a control device 220 that performs predetermined image processing on an imaging signal acquired by the endoscope 201 and collectively controls the operations of the entire endoscope system 200, and a display device 230 that displays the in-vivo image on which the image processing has been performed by the control device 220.

The endoscope 201 includes at least the lens unit 501 and the imaging unit 502 which have been described above.

The light source device 210 includes at least the first light source unit 31, the second light source unit 32, and the light source controller 33 which have been described above.

The control device 220 includes at least the communication module 91, the signal processing unit 92, the image processing unit 93, the input unit 94, the memory 95, the output unit 96, and the control unit 97 which have been described above.

According to the second embodiment described above, even the flexible endoscope system 200 can obtain the effects similar to those of the first embodiment described above.

### (Third embodiment)

Next, a third embodiment will be described. In the above first and second embodiments, application to the endoscope systems has been described, but in the third embodiment, application to a surgical microscope system will be described. Note that the same configurations as those of the endoscope system 1 according to the first embodiment described above are denoted by the same reference signs, and detailed description thereof will be omitted.

### [Configuration of surgical microscope system]

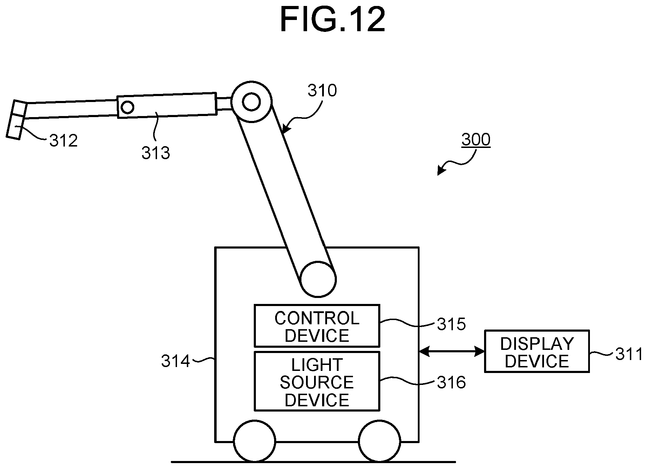

FIG. 12 is a diagram illustrating a schematic configuration of the surgical microscope system according to the third embodiment. A surgical microscope system 300 illustrated in FIG. 12 includes a microscope apparatus 310 that is a medical imaging device capturing and acquiring an image for observation of an object, and a display device 311 that displays the image captured by the microscope apparatus 310. Note that the display device 311 and the microscope apparatus 310 may be integrally configured.

The microscope apparatus 310 includes a microscope unit 312, a support portion 313, and a base portion 314. The microscope unit 312 captures a magnified image of a minute portion of the object, the support portion 313 includes an arm that is connected to a proximal end portion of the microscope unit 312 to turnably support the microscope unit 312, and the base portion 314 turnably holds a proximal end portion of the support portion 313 and is movable on a floor surface. The base portion 314 includes a control device 315 that controls the operation of the surgical microscope system 300, and a light source device 316 that generates white light, infrared light, or the like to be emitted from the microscope apparatus 310 to the object. Note that the control device 315 includes at least the communication module 91, the signal processing unit 92, the image processing unit 93, the input unit 94, the memory 95, the output unit 96, and the control unit 97 which have been described above. Furthermore, the light source device 316 includes at least the first light source unit 31, the second light source unit 32, and the light source controller 33 which have been described above. Furthermore, instead of being movably provided on the floor surface, the base portion 314 may be fixed on a ceiling, a wall surface, or the like to support the support portion 313.

The microscope unit 312 has, for example, a cylindrical shape, and internally includes the lens unit 501 and the imaging unit 502 which have been described above. The microscope unit 312 has a side surface provided with switches that receive inputs of operation instructions given to the microscope apparatus 310. The microscope unit 312 has an opening surface at a lower end, and the opening surface is provided with a cover glass (not illustrated) that protects the inside.

In the surgical microscope system 300 configured as described above, the user, such as an operator, holding the microscope unit 312 moves the microscope unit 312, performs zoom operation, or switches illumination light while variously operating the switches. Note that the microscope unit 312 preferably has a shape elongated in an observation direction so that the user can readily hold the microscope unit 312 and change a viewing direction. Therefore, the shape of the microscope unit 312 may be a shape other than the cylindrical shape, and may be, for example, a polygonal columnar shape.

According to the third embodiment described above, also in the surgical microscope system 300, the same effects as those of the first embodiment described above can be obtained.

### (Other embodiments)

Various aspects of the invention can be formed by appropriately combining a plurality of component elements disclosed in the medical observation system according to the first to third embodiments of the present disclosure which have been described above. For example, some component elements may be removed from all the component elements described in the medical observation system according to the first to third embodiments of the present disclosure described above. Furthermore, the component elements described in the medical observation system according to the first to third embodiments of the present disclosure described above may be appropriately combined.

The present disclosure is not limited to the embodiments, and in displaying the first image and the second image that are mixed in the specific region, a color obtained by blending (synthesizing) a display color of the second image and a color of the first image at a desired ratio can be set as the display color of the second image.

Specifically, the image processing unit 93 performs the following processing for each pixel, in color display of the group of second pixels in the specific region. It is also possible to display desired hue data (blended color) obtained by linearly complementing first image data (e.g., hue data) at a position overlapping each pixel of the second image and second image data (e.g., hue data) at each pixel of the second image by generating a color (synthetic data) of each pixel of the second image (group of second pixels).

Note that, as the desired ratio of the blended color, an appropriate ratio is allowed to be set by the image processing unit 93, according to the luminance or saturation of each pixel. Furthermore, settings can be changed according to the preference of an observer (operator). Furthermore, in the above example of "blending," "blending (synthesizing)" of the hue data has been described, but blending is not limited to the hue data, and at least one of luminance (brightness) data and saturation data of each pixel may be used for blending.

For example, the synthetic data may be generated by replacing the hue data with the luminance data so that the synthetic data may be displayed for each pixel of the second image (group of second pixels), as desired pixel data with luminance data.

Furthermore, the synthetic data may be generated by replacing the hue data with the saturation data so that the synthetic data may be displayed for each pixel of the second image (group of second pixels), as desired pixel data with saturation data.

Note that, in the above example of "blending," blending of the hue, luminance, and saturation data has been described, but the synthetic data may be generated by combining at least two of the hue, luminance, and saturation data so that the synthetic data may be displayed for each pixel of the second image (group of second pixels), as desired pixel data.

Furthermore, in the medical observation system according to the first to third embodiments of the present disclosure, the word "unit" which has been described above can be read as "means," "circuit," or the like. For example, the control unit can be read as control means or a control circuit.

Furthermore, programs executed by the medical observation system according to the first to third embodiments of the present disclosure are provided in the form of installable or executable file data and recorded in a computer-readable recording medium, such as a CD-ROM, flexible disk (FD), CD-R, digital versatile disk (DVD), USB medium, or flash memory.

Alternatively, the programs executed by the medical observation system according to the first to third embodiments of the present disclosure may be configured to be stored on a computer connected to a network such as the Internet and provided by being downloaded via the network.

It is noted that, in the description of the timing chart herein, a context of processes between timings has been clearly shown by using words, such as "first," "then," "subsequently," and the like, but the order of the processes necessary to carry out the present disclosure is not uniquely defined by these words. In other words, the order of the processes in the timing chart described herein may be changed within a consistent range.

Some embodiments of the present application have been described in detail with reference to the drawings, but these are provided by way of examples, and it is possible to carry out the present invention in other forms, including the modes described in the present disclosure, to which various modifications and improvements can be made on the basis of the knowledge of those skilled in the art.

Note that the present technology can also have the following configurations.

### (Supplementary note 1)

A medical image processing device including
an image processing unit that
uses
a first image based on first image data input from outside, and
a second image based on second image data input from outside, having an enhanced specific region of the first image,
and generates third image data in which the specific region of the first image is replaced with mixed image data having a mixture of a group of first pixels located in the specific region of the first image and a group of second pixels located in the specific region of the second image.

### (Supplementary note 2)

The medical image processing device according to (Supplementary note 1), in which
the image processor
generates the third image data by regularly arranging the group of first pixels and the group of second pixels in the specific region.

### (Supplementary note 3)

The medical image processing device according to (Supplementary note 2), in which
the image processor
generates the third image data by alternately arranging pixels of the group of first pixels and the group of second pixels, on horizontal lines in the specific region.

### (Supplementary note 4)

The medical image processing device according to (Supplementary note 2), in which
the image processor
generates the third image data by arranging pixels of the group of first pixels and the group of second pixels in a lattice pattern in the specific region.

### (Supplementary note 5)

The medical image processing device according to (Supplementary note 2), in which
the image processor
generates the third image data by alternately arranging the group of first pixels and the group of second pixels for each horizontal line or each vertical line in the specific region.

### (Supplementary note 6)

The medical image processing device according to (Supplementary note 1), in which
the image processor
generates the third image data by changing a display area of a second pixel in a mixed image based on the mixed image data according to a luminance value of each of the groups of second pixels.

### (Supplementary note 7)

The medical image processing device according to (Supplementary note 1), in which
the image processor
generates the third image data by irregularly arranging the group of first pixels and the group of second pixels in the specific region.

### (Supplementary note 8)

The medical image processing device according to any of (Supplementary note 1) to (Supplementary note 7), in which
the image processor
generates the third image data by converting color of the group of second pixels into a color to enhance the group of second pixels.

### (Supplementary note 9)

The medical image processing device according to any of (Supplementary note 1) to (Supplementary note 7), in which
the image processor
generates the third image data by converting color of each of the groups of second pixels into a color enhancing each of the groups of second pixels while maintaining a luminance value thereof.

### (Supplementary note 10)

The medical image processing device according to any of (Supplementary note 1) to (Supplementary note 7), in which
the image processor
generates the third image data by converting color of each of the groups of second pixels into a color according to a luminance value thereof.

### (Supplementary note 11)

The medical image processing device according to any of (Supplementary note 1) to (Supplementary note 7), in which
the image processor has a function of generating, for each pixel, synthetic data in which the first image data of each pixel of the group of first pixels is blended with the second image data of each pixel of the group of second pixels, as display data of each pixel of the group of second pixels.

### (Supplementary note 12)

The medical image processing device according to (Supplementary note 11), in which
the synthetic data is generated by blending using data information of at least one of hue, luminance, and saturation of each pixel of the first image and second image.

### (Supplementary note 13)

The medical image processing device according to any of (Supplementary note 1) to (Supplementary note 12), in which
the specific region is
a light-emitting region that emits light in response to irradiation of a fluorescent substance with excitation light.

### (Supplementary note 14)

A medical observation system including:
the medical image processing device according to any of (Supplementary note 1) to (Supplementary note 13);
a light source device that is configured to emit white light and near-infrared light to a subject where a fluorescent substance is sprayed; and
an observation device that generates the first image data by receiving reflection light from the subject when the white light is emitted, and generates the second image data by receiving light emitted from the fluorescent substance when the near-infrared light is emitted,
in which the medical image processing device
acquires the first image data and the second image data from the observation device.

### Reference Signs List

- 1, 200: ENDOSCOPE SYSTEM

- 2,: 202 INSERTION SECTION
- 3, 210, 316: LIGHT SOURCE DEVICE
- 4: LIGHT GUIDE
- 5: CAMERA HEAD
- 6: FIRST TRANSMISSION CABLE
- 7, 230, 311: DISPLAY DEVICE
- 8: SECOND TRANSMISSION CABLE
- 9, 220, 315: CONTROL DEVICE
- 10: THIRD TRANSMISSION CABLE
- 21: EYEPIECE
- 31: FIRST LIGHT SOURCE UNIT
- 32: SECOND LIGHT SOURCE UNIT
- 33: LIGHT SOURCE CONTROLLER
- 51: OPERATION RING UNIT
- 52: INPUT UNIT
- 61: FIRST CONNECTOR
- 62: SECOND CONNECTOR
- 91, 503: COMMUNICATION MODULE
- 92: SIGNAL PROCESSING UNIT
- 93: IMAGE PROCESSING UNIT
- 94: INPUT UNIT
- 95: MEMORY
- 96: OUTPUT UNIT
- 97: CONTROL UNIT
- 201: ENDOSCOPE
- 300: SURGICAL MICROSCOPE SYSTEM
- 310: MICROSCOPE APPARATUS
- 312: MICROSCOPE UNIT
- 313: SUPPORT PORTION
- 314: BASE PORTION
- 501: LENS UNIT
- 502: IMAGING UNIT
- 503: COMMUNICATION MODULE
- 504: CAMERA HEAD MEMORY
- 505: CAMERA HEAD CONTROLLER

## Claims

1. A medical image processing device comprising an image processor configured to:
use a first image based on first image data input from outside, and a second image based on second image data input from outside, the second image data including an enhanced specific region of the first image, and
generate third image data in which the specific region of the first image is replaced with mixed image data including a mixture of a group of first pixels located in the specific region of the first image and a group of second pixels located in the specific region of the second image.

2. The medical image processing device according to claim 1, wherein the image processor is configured to generate the third image data by regularly arranging the group of first pixels and the group of second pixels in the specific region.

3. The medical image processing device according to claim 2, wherein the image processor is configured to generate the third image data by alternately arranging pixels of the group of first pixels and the group of second pixels, on horizontal lines in the specific region.

4. The medical image processing device according to claim 2, wherein the image processor is configured to generate the third image data by arranging pixels of the group of first pixels and the group of second pixels in a lattice pattern in the specific region.

5. The medical image processing device according to claim 2, wherein the image processor is configured to generate the third image data by alternately arranging the group of first pixels and the group of second pixels for each horizontal line or each vertical line in the specific region.

6. The medical image processing device according to claim 1, wherein the image processor is configured to generate the third image data by changing a display area of a second pixel in a mixed image based on the mixed image data according to a luminance value of each of the groups of second pixels.

7. The medical image processing device according to claim 1, wherein the image processor is configured to generate the third image data by irregularly arranging the group of first pixels and the group of second pixels in the specific region.

8. The medical image processing device according to claim 1, wherein the image processor is configured to generate the third image data by converting color of the group of second pixels into a color to enhance the group of second pixels.

9. The medical image processing device according to claim 1, wherein the image processor is configured to generate the third image data by converting color of each of the groups of second pixels into a color enhancing each of the groups of second pixels while maintaining a luminance value thereof.

10. The medical image processing device according to claim 1, wherein the image processor is configured to generate the third image data by converting color of each of the groups of second pixels into a color according to a luminance value thereof.

11. The medical image processing device according to claim 1, wherein the image processor is configured to generate, for each pixel, synthetic data in which the first image data of each pixel of the group of first pixels is blended with the second image data of each pixel of the group of second pixels, as display data of each pixel of the group of second pixels.

12. The medical image processing device according to claim 11, wherein the synthetic data is generated by blending using data information of at least one of hue, luminance, and saturation of each pixel of the first image and second image.

13. The medical image processing device according to claim 1, wherein the specific region is a light-emitting region that emits light in response to irradiation of a fluorescent substance with excitation light.

14. A medical observation system comprising:
the medical image processing device according to claim 1;
a light source device configured to emit white light and near-infrared light to a subject where a fluorescent substance is sprayed; and
an observation device configured to
generate the first image data by receiving reflection light from the subject in a case where the white light is emitted, and
generate the second image data by receiving light emitted from the fluorescent substance when the near-infrared light is emitted,
wherein the medical image processing device is configured to acquire the first image data and the second image data from the observation device.
